Europäisches Patentamt

European Patent Office

Office européen des brevets

(11) Publication number: **0 502 418 A1**

## EUROPEAN PATENT APPLICATION

(21) Application number: **92103337.9**

(22) Date of filing: **26.02.92**

(51) Int. Cl.⁵: **A61K 31/275**

(30) Priority: **26.02.91 US 661581**

(43) Date of publication of application:
**09.09.92 Bulletin 92/37**

(84) Designated Contracting States:
**AT BE CH DE DK ES FR GB GR IT LI LU NL PT SE**

(71) Applicant: **BASF CORPORATION**
**8 Campus Drive**
**Parsippany, New Jersey 07054(US)**

(72) Inventor: **Rezvani, Amir H.**
**2407 Honeysuckle Road**
**Chapel Hill, North Carolina 27514(US)**
Inventor: **O'Brien, Robert A.**
**251 Vreeland Avenue**
**Nutley, New Jersey 07110(US)**

(74) Representative: **Karau, Wolfgang, Dr. et al**
**BASF Aktiengesellschaft Carl-Bosch-Strasse 38**
**W-6700 Ludwigshafen(DE)**

(54) **Use of levemopamil for the manufacture of a medicament for the treatment of alcohol abuse.**

㊼ The use of levemopamil and/or physiologically acceptable salts thereof for the treatment of alcohol dependency is described.

FIG.1

Rank Xerox (UK) Business Services

The present invention relates to the use of levemopamil for the treatment of alcohol abuse, and more particularly to the attenuation of alcohol preference, consumption and the effects of alcohol intoxication and withdrawal.

The incidence of alcohol dependence has been increasing throughout the world for many years. Some estimates indicate that there are at least five million alcohol dependent persons in the United States alone and another seven million with drinking problems. Alcoholism or alcohol dependence is generally characterized by habitual, compulsive, long term heavy consumption of alcohol and the development of withdrawal symptoms when drinking has stopped.

The treatment for alcoholism generally involves complete abstinence and detoxification. During this period of abstinence or withdrawal, physical symptoms including hallucinations, seizures and delirium tremens often appear. To alleviate some of the physical symptoms, tranquilizers and/or vitamins, if the patient is deficient, are prescribed. However, this treatment does not alleviate the desire for alcohol or the effects of intoxication.

Verapamil, a calcium channel blocking agent has been investigated as a treatment for alcohol dependency. See, for example, Rezvani et al., Prog. Neuro-Psychopharmacol. & Biol. Psychiat., 14: 623-631 (1990). However, verapamil does not have very effective cerebral availability or blood-brain permeability. See, Hofmann, et al., Arzneim.-Forsch./Drug Res., 39/(1): 3,304-308 (1989) and Szabo, Arzneim.-Forsch./Drug Res., 39/(1): 3,309-314 (1989).

Another calcium channel blocker, levemopamil [2-isopropyl-5-(methylphenethylamino)-2-phenylvaleronitrile], also known as emopamil and (S)-emopamil, has been used to treat hypoxic tissue damage, oxygen deficiency states of the brain and migraine headaches. See, for example, U.S. Patents 4,596,820 and 4,914,125, and EP 147,707, all of which are hereby incorporated by reference.

In spite of these disclosures, there remains a need for an effective treatment of alcohol dependency, including the attenuation of the effects of alcohol preference, consumption, intoxication and withdrawal.

We have found that a compound having strong calcium antagonist and serotonin antagonist properties, and/or the physiologically acceptable salts thereof, causes an attenuation of alcohol dependence, including alcohol preference, consumption, intoxication and withdrawal. Specifically, levemopamil has been found to be particularly effective in the treatment of alcohol dependence.

It is an object of the present invention to provide a method of treatment of alcohol dependency by administration of an effective dose of a compound having calcium and serotonin antagonist properties and/or a physiologically acceptable salt thereof.

It is another object of the present invention to provide a method of prophylaxis or treatment of alcohol dependency by administration of an effective dose of levemopamil and/or a physiologically acceptable salt thereof.

These and other objects of the present invention will be more fully understood from the following description of the invention with reference to the illustrations appended hereto.

Figure 1 shows a comparison of ethanol intake of alcohol preferring rats which have been administered levemopamil or a control vehicle.

Figure 2 shows a comparison of water intake of alcohol preferring rats which have been administered levemopamil or a control vehicle.

Figure 3 shows a comparison of total fluid intake of alcohol preferring rats which have been administered levemopamil or a control vehicle.

Figure 4 shows a comparison of food intake of alcohol preferring rats which have been administered levemopamil or a control vehicle.

Figure 5 shows the effects of ethanol intake of alcohol preferring rats which have been administered various dosages of levemopamil or a control vehicle.

Figure 6 shows the effects of water intake of alcohol preferring rats which have been administered various dosages of levemopamil or a control vehicle.

Figure 7 shows a comparison of ethanol intake of alcohol preferring rats which have been administered nimodipine or a control vehicle.

Figure 8 shows a comparison of water intake of alcohol preferring rats which have been administered nimodipine or a control vehicle.

Figure 9 shows a comparison of total fluid intake of alcohol preferring rats which have been administered nimodipine or a control vehicle.

Figure 10 shows a comparison of food intake of alcohol preferring rats which have been nimodipine or a control vehicle.

We have found that compounds having calcium and serotonin antagonist properties and/or the physiologically acceptable salts thereof, cause an attenuation of alcohol preference, consumption, intoxication and withdrawal. Levemopamil is a strong phenylalkylamine calcium and serotonin antagonist, and is particularly preferred.

As used herein, the expression "alcohol dependency" means a patient physically, emotionally and/or psychologically dependent upon alcohol. The terms alcohol dependency, alcohol abuse and alcoholism are interchangeable. The term "intoxication" means the result of drinking an excessive amount of alcohol over a relatively short period of time. Intoxication generally depresses the activity of the central nervous system, leading to loss of normal mental and physical control. As used herein, the expression "treating alcohol dependency" means an attenuation of alcohol dependence, including an attenuation of alcohol preference and consumption, and an attenuation of the effects of intoxication and withdrawal symptoms.

While not wishing to be bound by any particular theory, it is believed that levemopamil and/or the physiologically acceptable salts thereof have an significant anti-serotonin action which reaches the potency of known serotonin antagonists. The cerebral availabilic of levemopamil is significantly higher than that of the comparison compound verapamil. In addition, it has been shown by means of the brain uptake index (BUI) that levemopamil passes the blood/brain barrier more rapidly and in considerably larger amounts than does verapamil.

By reason of its excellent serotonin-antagonistic action and strong calcium channel blocking capability and its high cerebral availability, levemopamil is particularly suitable for the prophylaxis and treatment of alcohol dependency.

Levemopamil may be produced by the method described in U.S. Patent 4,596,820, hereby incorporated by reference.

Levemopamil can be administered in a conventional manner orally, parenterally (intravenously, intramuscularly, subcutaneously) or rectally.

Examples of suitable physiologically tolerated acids include, for example, hydrochloric acid, hydrobromic acid, sulfuric acid, phosphoric acid, acetic acid, maleic acid, lactic acid tartaric acid, citric acid and fumaric acid.

The dosage depends on the age, condition and weight of the patient and on the mode of administration. As a rule, the daily dose of active substance is from about 1 to 50 mg/kg body weight on oral and rectal administration and from about 0.1 to 5 mg/kg body weight on parenteral administration.

Levemopamil can be used in conventional solid or liquid pharmaceutical forms for administration, for example as tablets, film-coated tablets, sugar-coated tablets, capsules, powders, granules, suppositories or solutions. These are prepared in a conventional manner. The active substances can be processed in this connection with the conventional pharmaceutical aids such as tablet binders, fillers, preservatives, tablet disintegrants, flow-regulating agents, plasticizers, wetting agents, dispersants, emulsifiers, suppository bases, solvents, retardants and/or antioxidants. The forms for administration obtained in this way normally contain the active substance in an amount of from 1 to 99 percent by weight.

EXAMPLES

EXAMPLE 1

Tablets of the following composition are produced in a tableting machine in a conventional manner:

| 40 mg | Levemopamil hydrochloride |
|---|---|
| 120 mg | corn starch |
| 13.5 mg | gelatin |
| 45 mg | lactose |
| 2.25 mg | Aerosil® (chemically pure silica in submicroscopically fine distribution) |
| 6.75 mg | potato starch (as 6% strength paste). |

EXAMPLE 2

Sugar-coated tablets of the following composition are produced in a conventional manner:

3

| 20 mg | Levemopamil hydrochloride |
| 60 mg | core composition |
| 60 mg | sugar-coating composition |

The core composition comprises 9 parts of corn starch, 3 parts of lactose and 1 part of Luviskol. VA 64 (60:40 vinylpyrrolidone/vinyl acetate copolymer). The sugar-coating composition comprises 5 parts of sucrose, 2 parts of corn starch, 2 parts of calcium carbonate and 1 part of talc. The sugar-coated tablets produced in this wire are then provided with an enteric coating.

EXAMPLE 3

10 g of levemopamil hydrochloride are dissolved in 5000 ml of water with the addition of NaCl, and the pH is adjusted to 6.0 with 0.1 N NaOH so that a solution which is isotonic with blood is produced. 5 ml portions of this solution are dispensed into ampoules and sterilized.

EXAMPLE 4

Suppositories of the following composition are produced in a conventional manner:

| 100 mg | Levemopamil hydrochloride |
| 40 mg | Aerosil® silica |
| 1900 mg | hard fat. |

EXAMPLE 5

Levemopamil was administered in eight selectively-bred adult male alcohol preferring Wistar rats (hereinafter "P") obtained from the Indiana Alcohol Research Center at the University of Indiana School of Medicine.

These lines of rats have been developed genetically through selected breeding by Lumeng et al., New strains of rats with alcohol preference and nonpreference. In: Thurman et al., Eds. Alcohol and Aldehyde Metabolizing Systems. Vol. 3. New York: Academic Press, 1977. pp. 537-544. The P rats prefer to drink at least 5g/Kg body weight/day of a 10% (v/v) ethanol solution in a free-choice situation with food and water available. These rats have been widely used as an animal model of alcoholism. See, Tabakoff et al., Biochemical Pharmacology of Alcohol. Meltzer, Ed. In: Psychopharmacology: The Third Generation of Progress. New York: Raven Press, 1978. pp.1521-1526.

The criteria for classification of P rats was that the average daily intake of ethanol (10%v/v) in P rats should be equal or greater than 5g/Kg body weight/day and the ethanol to water intake ratio should be equal or greater than 2:1.

After establishing a stable baseline for alcohol and water intake, Each animal was injected twice (9:30 and 16:30 hours) with a 10 mg/Kg dosage of levemopamil or an equal volume of the vehicle. Water, alcohol and food intake were monitored.

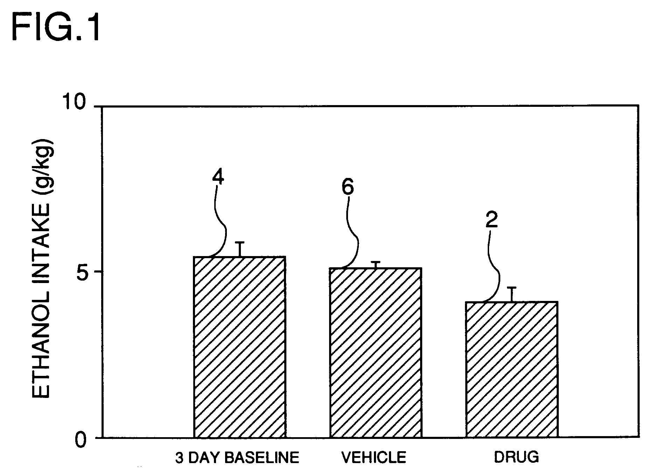

As shown in Figure 1, the administration of 10 mg/Kg of levemopamil significantly attenuated alcohol intake or consumption (2) in view of the baseline consumption (4) and also in view of the rats administered the vehicle alone (6).

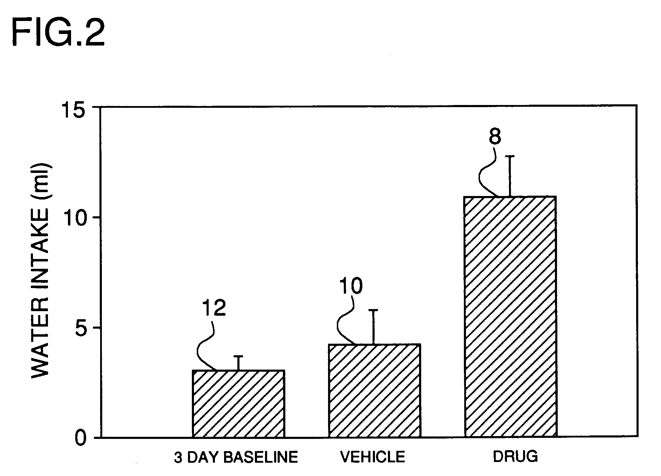

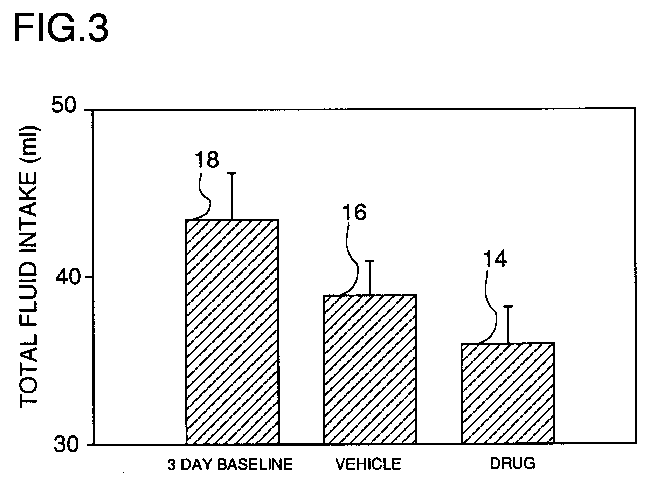

Figure 2 shows that the rats administered levemopamil displayed significantly increased water intake (8) than those rats administered the vehicle (10) and also over the base-line established (12). Total fluid intake was decreased in the rats administered emopamil (14) in comparison to those rats administered the vehicle alone (16) and to the base-line intake (18), as illustrated in Figure 3. These results show that the animals treated with levemopamil significantly reduced the volume of alcohol intake and switched their preference from alcohol to water.

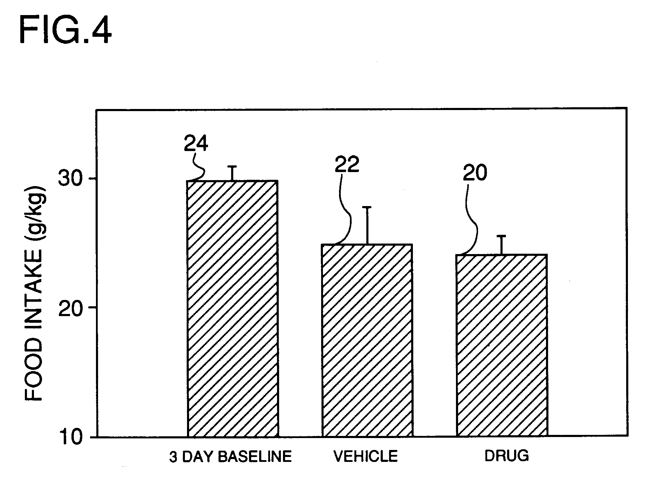

Figure 4 shows that food intake was unaffected by the administration of levemopamil (20) or the vehicle (22) but slightly decreased over the base-line intake (24). Figure 4 shows that the administration exerts a specific effect only on alcohol intake and not other consummatory behavior.

EXAMPLE 6

Levemopamil and a vehicle were administered in eight P rats obtained from the Indiana Alcohol Research Center at the University of Indiana School of Medicine, as described in Example 5. After establishing a stable baseline for alcohol and water intake, Each animal was injected twice (9:15 and 16:00 hours) with either one of three doses of levemopamil (3.3, 10, and 20 mg/Kg) or an equal volume of the vehicle (a solution of 1 ml absolute ethanol and 4 ml distilled water. Water and alcohol intake were monitored. The amount of alcohol and water intake was recorded twice daily before each administration of vehicle or levemopamil and continued during the drug washout periods.

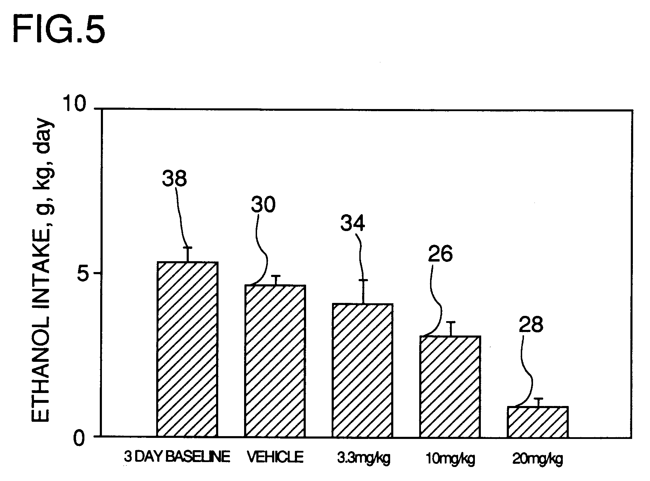

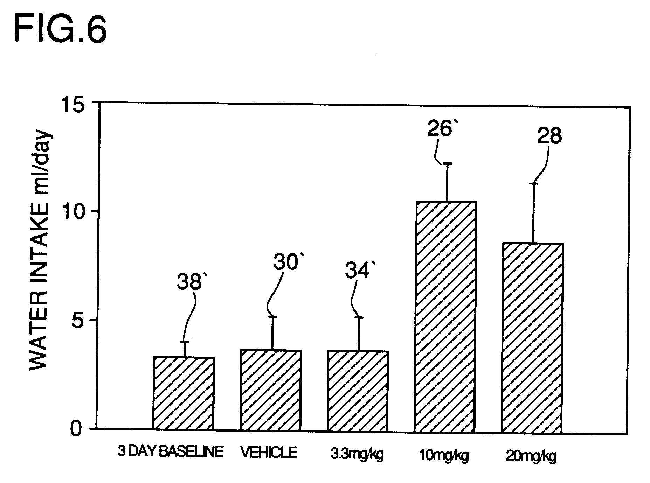

P rats with free access to alcohol and water, consumed an average of 5.05±1,3 g/Kg of ethanol and 2.7±1.05 ml/Kg of water per day. As shown in Figures 5 and 6, administration of 10mg/Kg (26, 26') and 20 mg/Kg (28, 28') of levemopamil twice daily over a period of one day, as compared to the vehicle (30, 30') and the base-line (32, 32'), significantly reduced the intake of ethanol and significantly increased the intake of water. Injection of a low dose (3.3mg/Kg) of levemopamil (34, 34') did not induce a significant change in ethanol or water intake. Alcohol intake returned to base-line levels during the drug washout periods.

EXAMPLE 7

Nimodipine was administered in ten P rats obtained from the Indiana Alcohol Research Center at the University of Indiana School of Medicine, as described in Example 5.

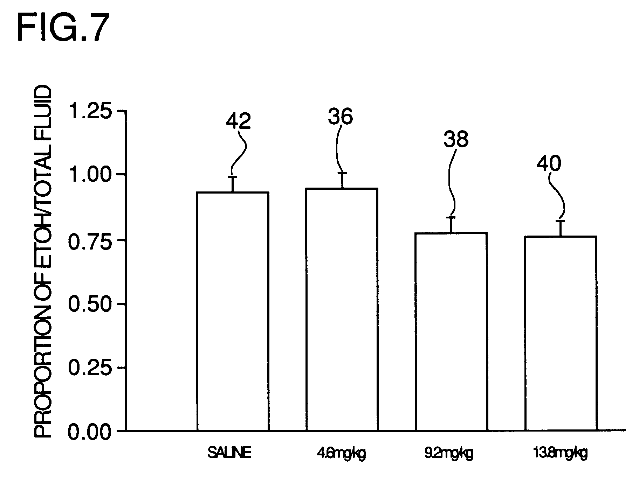

Three doses of nimodipine (4.6, 9.2 and 13.8 mg/Kg) or an equal volume of the vehicle (saline) were administered to the animals. Water, alcohol and food intake were monitored. Figure 7 shows that nimodipine failed to exert a significant effect on ethanol intake at any dose [4.6 mg/Kg (36), 9.2 mg/Kg (38 or 13.8 mg/Kg (40)], using saline as a control (42).

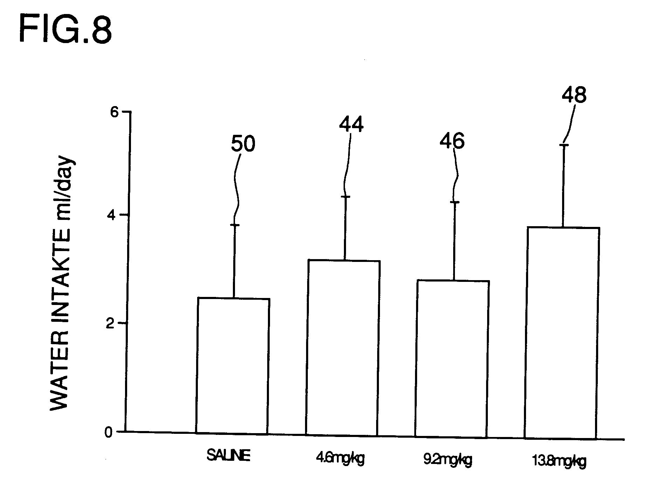

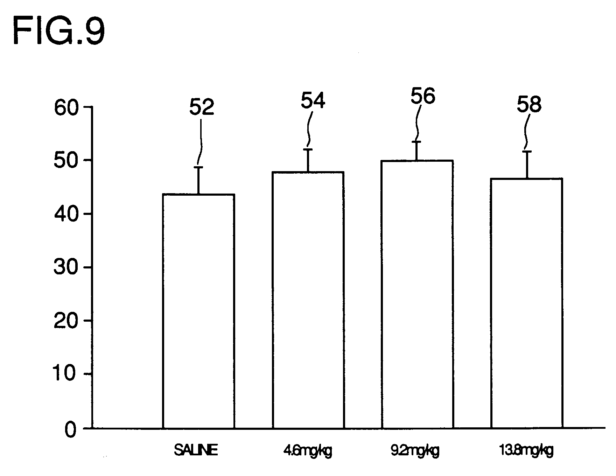

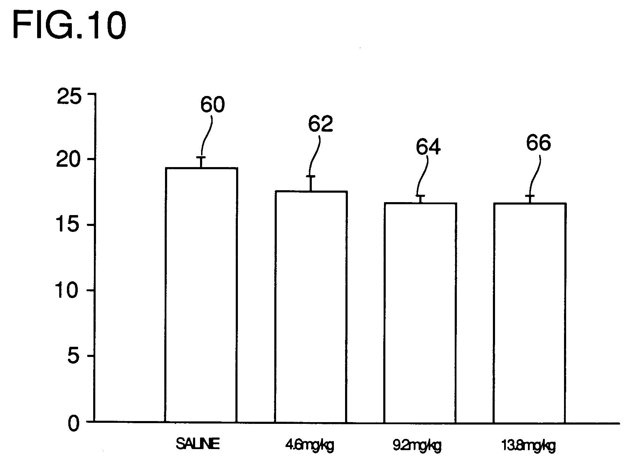

Figure 8 shows that water intake was not significantly affected by the administration of nimodipine at any dose [4.6 mg/Kg (44), 9.2 mg/Kg (46) or 13.8 mg/Kg (48)], using saline as the control (50). Figure 9 shows that a comparison of total fluid intake of the control (52) and administration of varied doses of nimodipine [4.6 mg/Kg (54), 9.2 mg/Kg (56) or 13.8 mg/Kg (58)], was unaffected by nimodipine, as was a comparison of the control (60) and food intake [4.6 mg/Kg (62), 9.2 mg/Kg (64) or 13.8 mg/Kg (66)], as illustrated in Figure 10.

Whereas particular embodiments of the invention have been described above for purposes of illustration, it will be appreciated by those skilled in the art that numerous variations may be made without departing from the invention as described in the appended claims.

**Claims**

1. A method of treating alcohol dependency in patients suffering therefrom, which comprises administering to said patients an effective amount of a compound or a physiologically acceptable salt thereof, having effective calcium and serotonin antagonistic properties.

2. A method of treating alcohol dependency as claimed in Claim 1, wherein said compound is levemopamil or a physiologically acceptable salt thereof.

# FIG.1

# FIG.2

FIG.3

# FIG.4

# FIG.5

FIG.6

# FIG.7

# FIG.8

# FIG.9

# FIG.10

European Patent Office

EUROPEAN SEARCH REPORT

Application Number

EP    92 10 3337

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,Y | EP-A-0 444 854 (ELI LILLY AND COMPANY) 4 September 1991 * page 20, line 17 - page 20, line 25; claims 1,17 * | 1,2 | A61K31/275 |
| D,Y | ARZNEIMITTELFORSCHUNG/DRUG RESEARCH vol. 39(I), no. 3, 1989, AULENDORF pages 304 - 308; H.P.HOFFMANN ET AL: '(s)-emopamil, a novel calcium and serotonin antagonist for the treatment of cerebrovascular disorders' *summary; page 308, right column, paragraph 1* | 1,2 | |
| Y | MEDICAL HYPOTHESES vol. 29, no. 4, 1989, pages 237 - 239; J.BACKON: 'Predicting new effective treatments of alcohol addiction on the basis of their properties of inhibition of noradrenergic activity and/or thromboxane or on the activation of the dopamine reward system and /or Beta-endorphin' *abstract* | 1,2 | |
| Y | J. CLIN. PSYCHIATRY vol. 47, no. 4, 1986, pages 4 - 8; R.W.FULLER: 'Pharmacologic Modification of Serotonin Function: Drugs for the study and treatment of Psychiatric and other Disorders' *summary; page 6, left column, paragraph 3* | 1,2 | TECHNICAL FIELDS SEARCHED (Int. Cl.5) A61K |

The present search report has been drawn up for all claims

| Place of search | Date of completion of the search | Examiner |
|---|---|---|
| MUNICH | 02 JUNE 1992 | TZSCHOPPE D.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)